# EUROPEAN PATENT APPLICATION

(11) **EP 4 831 195 A1**
(43) Date of publication of application: **28.10.2026**
(21) Application number: 24906451.0
(22) Date of filing: 19.12.2024
(51) Int. Cl.: C07K 14/605, C07K 14/435, C07K 14/00, C07K 14/575, C07K 1/04, C07K 1/06, C07K 1/30

(54) **SYNTHETIC METHOD FOR TIRZEPATIDE**

(30) Priority: 20.12.2023 CN 202311761341
(71) Applicant: Hybio Pharmaceutical Co., Ltd., Shenzhen, Guangdong 518057 (CN)
(72) Inventor: YANG, Peng, Shenzhen, Guangdong 518057 (CN); WANG, Wei, Shenzhen, Guangdong 518057 (CN); YIN, Chuanlong, Shenzhen, Guangdong 518057 (CN); TANG, Yangming, Shenzhen, Guangdong 518057 (CN); YU, Pinxiang, Shenzhen, Guangdong 518057 (CN)
(74) Representative: Fabry, Bernd
(86) International application number: PCT/CN2024/140569
(87) International publication number: WO 2025/130972

(57) **Abstract**

The present disclosure provides a synthesis method for tirzepatide, and the method of the present disclosure avoids the use of complex starting building blocks. The specially modified building blocks used include Fmoc-Lys(AEEA-AEEA-γ-Glu-eicosanedioic acid)-OH, Fmoc-Ile-Aib-OH, and Boc-Tyr-Aib-OH. The use of these simple building blocks can not only ensure the condensation efficiency but also effectively control the fragment quality, and finally, a final product can be obtained with a relatively high content yield.

## Description

### TECHNICAL FIELD

The present disclosure relates to the field of pharmaceutical chemistry, and in particular to a synthesis method for tirzepatide.

### BACKGROUND

Tirzepatide is a GLP-1 and GIP dual-target agonist developed by Eli Lilly and Company. It was approved by the FDA on May 13, 2022, and marketed in the United States.

Its structure (peptide sequence) is:
NH₂-Tyr-Aib-Glu-Gly-Thr-Phe-Thr-Ser-Asp-Tyr-Ser-Ile-Aib-Leu-Asp-Lys-Ile-Ala-Asn-Lys(AEEA-AEEA-γ-Glu-eicosanedioic acid)-Ala-Phe-Val-Asn-Trp-Leu-Ile-Ala-Gly-Gly-Pro-Ser-Ser-Gly-Ala-Pro-Pro-Pro-Ser-CONH₂
wherein AEEA is a special amino acid: 2-(2-(2-aminoethoxy)ethoxy)acetic acid.

Specifically, it is shown in the figure below:

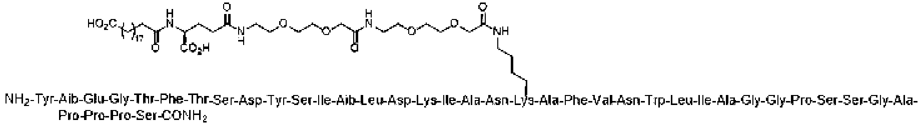

The existing synthesis processes of tirzepatide are roughly divided into two major categories: **The first category is a liquid-phase splicing mode**: Eli Lilly and Company uses a liquid-phase splicing mode to perform the synthesis of tirzepatide in the patent with application number CN113330024A. The four fully protected fragments for the liquid-phase splicing are fragment (1-14), fragment (15-21), fragment (22-29), and fragment (30-39), respectively. Fragment (22-29) and fragment (30-39) are spliced through a liquid phase and treated using a nanofiltration device to obtain fragment (22-39); immediately thereafter, fragment (22-39) undergoes a liquid-phase condensation with fragment (15-21) and a nanofiltration treatment to obtain fragment (15-39); finally, fragment (15-39) undergoes a liquid-phase condensation with fragment (1-14), followed by deprotection to obtain a crude peptide. In this process, the quality of the four fully protected fragments is difficult to control, especially for the identification of racemic impurities. In addition, the purification of intermediates in the liquid-phase reaction is not easy to perform. **The second category is an Fmoc solid-phase synthesis mode**. This category of synthesis methods has been widely reported, including sequentially performing a standard Fmoc solid phase and removing a protecting group of Lys to continue side chain coupling synthesis. There is also a fragment condensation.
1) Patent CN113330024A: synthesizes tirzepatide by adopting a standard Fmoc solid-phase synthesis strategy, wherein the Lys side chain at position 20 adopts ivDde for protection. Hydrazine hydrate is used to selectively remove Lys at position 20, followed by connecting (AEEA)₂-γ-Glu-C₂₀diacid, and finally, cleavage and deprotection are performed to obtain a crude peptide. The limitation of this method lies in the fact that the coupling time is too long; a conventional amino acid generally adopts a condensation time of 4-8 h, and for a special amino acid, the condensation time is even as long as 18 h.
2) Patents CN112592387B and CN114736271A use a serine-containing pseudoproline dipeptide or Dmb-protected glycine as specially modified building blocks, and performing stepwise coupling can effectively inhibit shrinkage during peptide resin synthesis. However, the Dmb-containing glycine will cause dipeptide deletion at this site and the next site after condensation.
3) Patents CN115651075B and CN110903355A use fragment (1-4) as a whole unit, intending to solve the problem of difficult coupling of the amino acids at positions 1 and 2. However, since fragment (1-4) must be prepared via additional solid-phase or liquid-phase synthesis, this approach is costly and not conducive to large-scale production.
4) Patents CN116120403A and CN115991742A use large fragments such as (1-16), (17-26), (27-39) or (1-14), and (22-29) as splicing units. The disadvantage of this type of methods is that extra synthesis procedures are required for large fragments with a relatively large usage amount of fragment peptides. Apart from cost issues, quality control difficulties also exist for fully protected large fragments.
5) Patents CN115181173A, CN115181174A, and CN115160429A use a 2- to 6-peptide or a 5- to 7-peptide containing a special site, which can partially solve the deletion problem. However, its shortcomings are also quite significant. Firstly, the usage amount of the fragment peptides is relatively large, and secondly, there are issues of cost and quality control of the fragment peptides.

### SUMMARY

In order to solve the problems of low efficiency, low purity, difficult acquisition of raw materials, and high purification difficulty of tirzepatide synthesis in the prior art, the present disclosure provides a method for synthesizing tirzepatide by a solid-phase stepwise coupling strategy. The method of the present disclosure avoids the use of complex starting building blocks. The specially modified building blocks used include Fmoc-Lys(AEEA-AEEA-γ-Glu-eicosanedioic acid)-OH, Fmoc-Ile-Aib-OH and Boc-Tyr-Aib-OH. The use of these simple building blocks can not only ensure the condensation efficiency but also effectively control the fragment quality, and finally, a final product can be obtained with a relatively high content yield.

The synthetic route of the present disclosure is shown below:

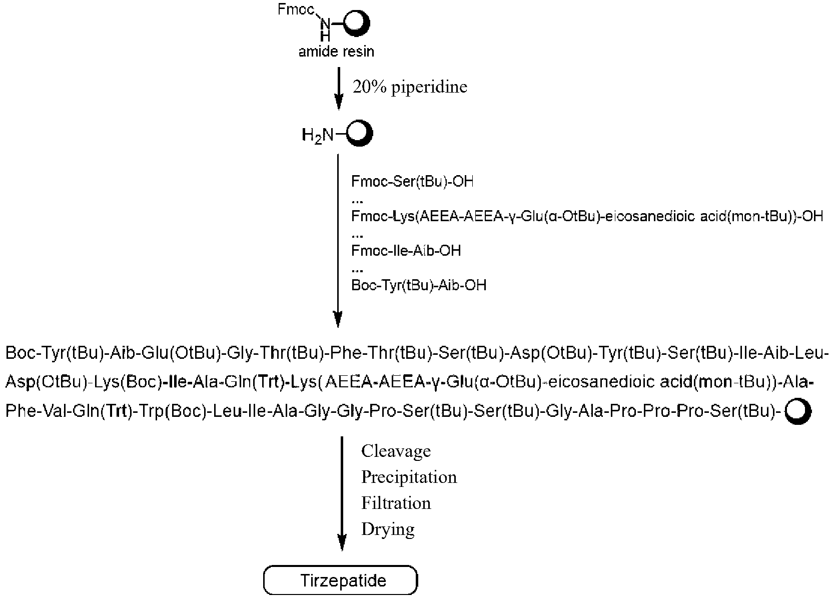

Specifically, the present disclosure provides a method for solid-phase synthesis of tirzepatide, comprising the following steps:
S1) employing an amide resin as the solid support, and adopting an Fmoc solid-phase synthesis strategy to sequentially couple Fmoc-protected amino acids or specially modified building blocks to afford a fully protected tirzepatide peptide resin; and
S2) subjecting the resulting fully protected tirzepatide peptide resin to cleavage, precipitation, filtration, and drying to afford tirzepatide;
wherein the sequential coupling of Fmoc-protected amino acids or specially modified building blocks comprises sequential coupling of Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Ala-OH, Fmoc-Ile-OH, Fmoc-Leu-OH, Fmoc-Trp(Boc)-OH, Fmoc-Gln(Trt)-OH, Fmoc-Val-OH, Fmoc-Phe-OH, Fmoc-Ala-OH, Fmoc-Lys(AEEA-AEEA-γ-Glu(α-OtBu)-eicosanedioic acid(mon-tBu))-OH, Fmoc-Gln(Trt)-OH, Fmoc-Ala-OH, Fmoc-Ile-OH, Fmoc-Lys(Boc)-OH, Fmoc-Asp(OtBu)-OH, Fmoc-Leu-OH, Fmoc-Ile-Aib-OH, Fmoc-Ser(tBu)-OH, Fmoc-Tyr(tBu)-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, and Boc-Tyr-Aib-OH.

Further, the Fmoc solid-phase synthesis strategy in step S1) comprises:
S11) removing an Fmoc protecting group; and
S12) coupling of Fmoc-protected amino acids or specially modified building blocks; wherein in S11), 20% piperidine/DMF is employed as an Fmoc deprotection reagent; and said 20% piperidine/DMF is a mixed solution of piperidine and DMF at a volume ratio of 1:4;
the coupling the Fmoc-protected amino acids or specially modified building blocks in S12) is carried out in the presence of an amino acid or specially modified building block coupling agent.

Further, deprotection in S11) comprises adding the Fmoc deprotection reagent and maintaining the reaction for 5-10 min, and washing with DMF, adding the Fmoc deprotection reagent again and maintaining the reaction for 5-10 min, and washing with DMF.

Further, the coupling agent forthe protected amino acids is a combination of DIC and A or a combination of DIPEA, A, and B, wherein A is Oxyma, HOBt, or HOAt, and B is one of PyBOP, PyAOP, HATU, HBTU, and TBTU; preferably, the coupling agent is a composition of DIC and HOBt.

Further, when coupling the protected amino acids, the molar amount of the coupling agent is 1-1.5 times the molar amount of the amino acids.

Further, the coupling agent for coupling the specially modified building blocks Fmoc-Lys(AEEA-AEEA-γ-Glu(α-OtBu)-eicosanedioic acid(mon-tBu))-OH, Fmoc-Ile-Aib-OH, and Boc-Tyr(tBu)-Aib-OH is a combination of DIC, HOBt, and DIPEA.

Further, when coupling the specially modified building blocks, a molar ratio of the specially modified building blocks, DIC, HOBt and DIPEA is 1:(1-1.5):(1-1.5):(0.1-0.5), preferably 1:1.2:1.2:0.1.

Further, the amide resin in S1) is Rink amide resin, Rink amide AM resin, Rink amide MBHA resin, or Sieber resin. Among them, Rink amide MBHA resin and Sieber resin are preferred.

Further, a substitution level of the amide resin in S1) is 0.2-0.8 mmol/g, preferably 0.4-0.6 mmol/g.

Further, a reaction temperature in S1) is room temperature or 20-35 °C.

Further, the cleavage in S2) performed using a mixed solution of TFA, TIPS, EDT, and H₂O, and preferably, the volume ratio of TFA, TIPS, EDT and H₂O is 91:3:3:3.

Further, the precipitation mode in S2) comprises adding an excess of methyl tert-butyl ether into the cleavage reaction mixture to effect precipitation. Preferably, the added methyl tert-butyl ether is 5-10 times the volume of the cleavage reaction mixture.

| Further, the filtration mode in S2) is filter cloth filtration. Abbreviation | Full version |
|---|---|
| Fmoc | 9-Fluorenylmethyloxycarbonyl |
| HOBt | 1-Hydroxybenzotriazole |
| DIPEA | N,N-Diisopropylethylamine |
| PyBOP | (Benzotriazol-1-yloxy)tripyrrolidinophosphonium hexafluorophosphate |
| DMF | N,N-Dimethylformamide |
| TFA | Trifluoroacetic acid |
| EDT | Ethanedithiol |
| tBu | tert-Butyl |
| AEEA | 2-(2-(2-Aminoethoxy)ethoxy)acetic acid |
| Dmb | 2,4-Dimethoxybenzyl |
| TIPS | Triisopropylsilane |
| Trt | Trityl |
| Boc | tert-Butyloxycarbonyl |
| OtBu | tert-Butyl ester |
| eicosanedioic acid | Eicosanedioic acid |
| mon-tBu | mono-tert-Butyl ester |

### Beneficial Effects

1) The tirzepatide synthesis method of the present disclosure has simple synthesis steps and easily available starting materials. Through stepwise coupling, the use of large peptide fragments is avoided, and the quality of the starting materials is easy to control.
2) For specially modified building blocks that are difficult to couple, a small special fragment is used for synthesis, and meanwhile, adding a small amount of DIPEA can significantly improve the coupling efficiency.
3) The tirzepatide synthesis method of the present disclosure can finally synthesize tirzepatide with a relatively high content yield while maintaining relatively high purity, which has significant cost advantages and is favorable for large-scale synthesis.

### DESCRIPTION OF THE DRAWINGS

FIG. 1 shows a synthetic route of the present disclosure.
FIG. 2 shows the HPLC results of the tirzepatide crude peptide synthesized in Example 1.
FIG. 3 shows the HPLC results of the tirzepatide crude peptide synthesized in Comparative Example 1.

### DETAILED DESCRIPTION

To make the aforementioned objectives, features, and advantages of the present disclosure more apparent and comprehensible, specific embodiments are described in detail below, but are not to be construed as limiting the implementable range of the present disclosure.

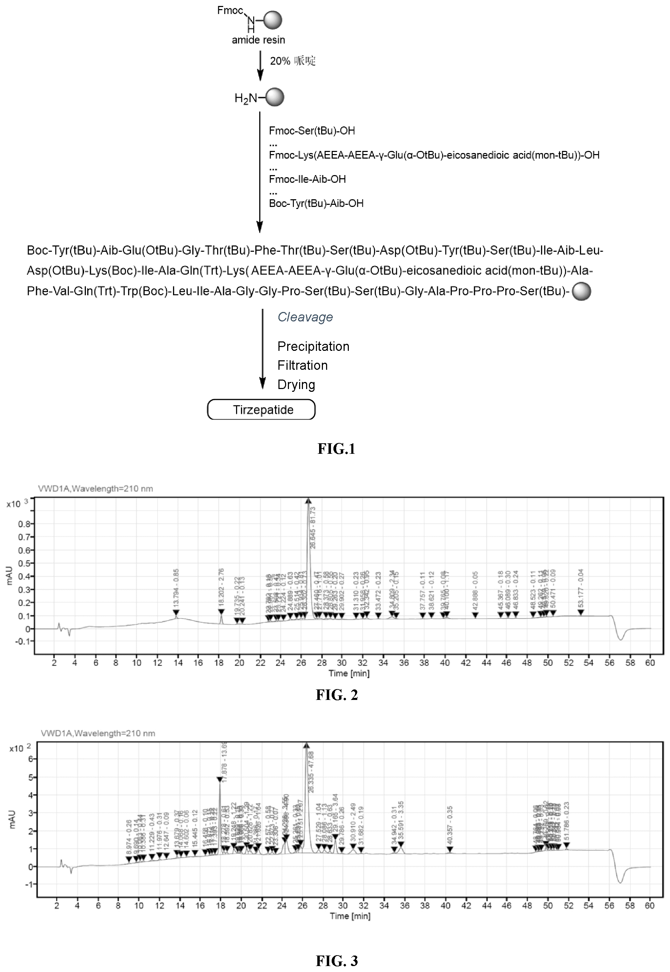

Description is made below in conjunction with FIG. 1. The present disclosure provides a solid-phase synthesis method for tirzepatide, which comprises the following steps:
S1) employing an amide resin as the solid support, and using an Fmoc solid-phase synthesis strategy to sequentially couple Fmoc-protected amino acids or specially modified building blocks, so as to obtain a fully protected tirzepatide peptide resin;
wherein the sequential coupling of Fmoc-protected amino acids or specially modified building blocks is: the sequential coupling of Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Ala-OH, Fmoc-Ile-OH, Fmoc-Leu-OH, Fmoc-Trp(Boc)-OH, Fmoc-Gln(Trt)-OH, Fmoc-Val-OH, Fmoc-Phe-OH, Fmoc-Ala-OH, Fmoc-Lys(AEEA-AEEA-γ-Glu(α-OtBu)-eicosanedioic acid(mon-tBu))-OH, Fmoc-Gln(Trt)-OH, Fmoc-Ala-OH, Fmoc-Ile-OH, Fmoc-Lys(Boc)-OH, Fmoc-Asp(OtBu)-OH, Fmoc-Leu-OH, Fmoc-Ile-Aib-OH, Fmoc-Ser(tBu)-OH, Fmoc-Tyr(tBu)-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, and Boc-Tyr-Aib-OH;
the method of the Fmoc solid-phase synthesis strategy in step S1) comprises:
   S11) removing an Fmoc protecting group, wherein 20% piperidine/DMF is used as an Fmoc deprotection reagent; the 20% piperidine/DMF is a mixed solution of piperidine and DMF in a volume ratio of 1:4; the method for deprotection comprises adding the Fmoc deprotection reagent to perform deprotection with a deprotection time of 5-10 min, and after washing with DMF, adding the Fmoc deprotection reagent again to perform deprotection for 5-10 min, and washing with DMF; and
   S12) coupling the Fmoc-protected amino acids or specially modified building blocks, wherein a coupling agent for coupling the protected amino acids is a composition of DIC and HOBt; wherein the usage amounts of both DIC and HOBt are 1.2 times the molar amount of the protected amino acids;
   the coupling agent for coupling the specially modified building blocks Fmoc-Lys(AEEA-AEEA-γ-Glu(α-OtBu)-eicosanedioic acid(mon-tBu))-OH, Fmoc-Ile-Aib-OH, and Boc-Tyr(tBu)-Aib-OH is a combination of DIC, HOBt, and DIPEA; when coupling the specially modified building blocks, a molar ratio of the specially modified building blocks, DIC, HOBt, and DIPEA is 1:1.2:1.2:0.1;
   S2) subjecting the synthesized fully protected tirzepatide peptide resin to cleavage, precipitation, filtration, and drying to obtain tirzepatide, wherein the cleavage adopts a mixed solution of TFA, TIPS, EDT, and H₂O, and a volume ratio of TFA, TIPS, EDT, and H₂O is 91:3:3:3; the precipitation mode in S2) is adding methyl tert-butyl ether of 8 times the system volume to the cleavage reaction system to perform precipitation; the filtration mode is filter cloth filtration.

Further, the method may further comprise a step of further purifying the synthesized tirzepatide.

### Example 1

23.3 g of Rink amide MBHA Resin (amino acid resin) with a substitution level of 0.43 mmol/g was selected and added to a reaction column. DMF was added to swell for 30 min, and the solution was drained. A 20% piperidine/DMF solution was added to remove Fmoc twice (10 min + 10 min). After the removal, washing was performed 5 times with DMF. Fmoc-Ser(tBu)-OH (11.5 g, 30 mmol) and HOBt (4.86 g, 36 mmol) were weighed and added to the reaction column. Dissolution was performed with DMF, and then DIC (5.63 mL, 36 mmol) was added. The mixture was reacted at room temperature for 1 h. After the reaction was completed, as detected by ninhydrin, suction filtration was performed, and washing was performed 5 times with an appropriate amount of DMF. The above procedures were repeated to perform the coupling of other amino acids and specially modified building blocks, wherein the reaction time of conventional amino acids was 1 h. When coupling the specially modified building blocks Fmoc-Lys(AEEA-AEEA-γ-Glu(α-OtBu)-eicosanedioic acid(mon-tBu))-OH, Fmoc-Ile-Aib-OH, and Boc-Tyr-Aib-OH, 0.2 eq. of DIPEA needed to be added to promote the reaction, and the coupling times were 3 h, 3 h, and 2 h, respectively. After the coupling was completed, methyl tert-butyl ether was added to shrink the resin, and finally, 91.5 g of a peptide resin was obtained: Boc-Tyr(tBu)-Aib-Glu(OtBu)-Gly-Thr(tBu)-Phe-Thr(tBu)-Ser(tBu)-Asp(OtBu)-Tyr(tBu)-Ser(tBu)-Ile-Aib-Leu-Asp(OtBu)-Lys(Boc)-Ile-Ala-Gln(Trt)-Lys(AEEA-AEEA-γ-Glu(α-OtBu)-eicosanedioic acid(mon-tBu))-Ala-Phe-Val-Gln(Trt)-Trp(Boc)- Leu-Ile-Ala-Gly-Gly-Pro-Ser(tBu)-Ser(tBu)-Gly-Ala-Pro-Pro-Pro-Ser(tBu)-Rink amide MBHA resin.

**The specific coupling conditions are as follows:**

| **No.** | **Amino acid** | **Coupling conditions (DMF as the reaction solvent)** |
|---|---|---|
| 1 | Fmoc-Ser(tBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 2 | Fmoc-Pro-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 3 | Fmoc-Pro-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 4 | Fmoc-Pro-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 5 | Fmoc-Ala-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 6 | Fmoc-Gly-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 7 | Fmoc-Ser(tBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 8 | Fmoc-Ser(tBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 9 | Fmoc-Pro-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 10 | Fmoc-Gly-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 11 | Fmoc-Gly-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 12 | Fmoc-Ala-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 13 | Fmoc-Ile-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 14 | Fmoc-Leu-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 15 | Fmoc-Trp(Boc)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 16 | Fmoc-Gln(Trt)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 17 | Fmoc-Val-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 18 | F moc-Phe-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 19 | Fmoc-Ala-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 20 | Fmoc-Lys(AEEA-AEEA-γ-Glu(α-OtBu)-eicosanedioic acid(mon-tBu))-OH | Fmoc removal: 20% piperidine/DMF, 10 min × 2, washing 5 times with DMF; Coupling: 2.0 eq. AA/2.4 eq. HOBt/2.4 eq. DIC/ 0.2 eq. DIPEA, 3 h, rt. |
| 21 | Fmoc-Gln(Trt)-OH | Fmoc removal: 20% piperidine/DMF, 10 min × 2, washing 5 times with DMF; Coupling: 3.0 eq. AA/3.6 eq. HOBt/3.6 eq. DIC, 1.5 h, rt. |
| 22 | Fmoc-Ala-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 23 | Fmoc-Ile-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 24 | Fmoc-Lys(Boc)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 25 | Fmoc-Asp(OtBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 26 | Fmoc-Leu-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 27 | Fmoc-Ile-Aib-OH | Fmoc removal: 20% piperidine/DMF, 10 min × 2, washing 5 times with DMF; Coupling: 2.0 eq. AA/2.4 eq. HOBt/2.4 eq. DIC/ 0.2 eq. DIPEA, 3 h, rt. |
| 28 | Fmoc-Ser(tBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 29 | Fmoc-Tyr(tBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 30 | Fmoc-Asp(OtBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 31 | Fmoc-Ser(tBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 32 | Fmoc-Thr(tBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 33 | Fmoc-Phe-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 34 | Fmoc-Thr(tBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 35 | Fmoc-Gly-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 36 | Fmoc-Glu(OtBu)-OH | Fmoc removal: 20% piperidine/DMF, 10 min × 2, washing 5 times with DMF; Coupling: 3.0 eq. AA/3.6 eq. HOBt/3.6 eq. DIC, 1 h, rt. |
| 37 | Boc-Tyr(tBu)-Aib-OH | Fmoc removal: 20% piperidine/DMF, 10 min × 2, washing 5 times with DMF; Coupling: 2.0 eq. AA/2.4 eq. HOBt/2.4 eq. DIC/ 0.2 eq. DIPEA, 2 h, rt. |

Cleavage step: The cleavage reagent adopted in this technique was TFA/TIDPS/EDT/H₂O = 91/3/3/3, and the volume of the cleavage reagent was 8 times the weight of the resin. Cleavage was performed at room temperature for 2 h. The resin was removed by filtration. Methyl tert-butyl ether was added to the cleavage solution. The addition amount of the methyl tert-butyl ether was 8 times that of the cleavage solution (V/V). Precipitation and filtration were performed, and the filter cake was dried to finally obtain 48.0 g of a crude peptide product with a yield of 99.8%, a purity of 81.73%, and a content of 77.4%. (FIG. 2)

### Comparative Example 1

23.3 g of Rink amide MBHA Resin (amino acid resin) with a substitution level of 0.43 mmol/g was selected and added to a reaction column. DMF was added to swell for 30 min, and the solution was drained. A 20% piperidine/DMF solution was added to remove Fmoc twice (10 min + 10 min). After the removal, washing was performed 5 times with DMF. Fmoc-Ser(tBu)-OH (11.5 g, 30 mmol) and HOBt (4.86 g, 36 mmol) were weighed and added to the reaction column. Dissolution was performed with DMF, and then DIC (5.63 mL, 36 mmol) was added. The mixture was reacted at room temperature for 1 h. After the reaction was completed, as detected by ninhydrin, suction filtration was performed, and washing was performed 5 times with an appropriate amount of DMF. The above procedures were repeated to perform the coupling of other amino acids. After the coupling was completed, methyl tert-butyl ether was added to shrink the resin, and finally, 72 g of a peptide resin was obtained:
Boc-Tyr(tBu)-Aib-Glu(OtBu)-Gly-Thr(tBu)-Phe-Thr(tBu)-Ser(tBu)-Asp(OtBu)-Tyr(tBu)-Ser(tBu)-Ile-Aib-Leu-Asp(OtBu)-Lys(Boc)-Ile-Ala-Gln(Trt)-Lys(AEEA-AEEA-γ-Glu(α-OtBu)-eicosanedioic acid(mon-tBu))-Ala-Phe-Val-Gln(Trt)-Trp(Boc)- Leu-Ile-Ala-Gly-Gly-Pro-Ser(tBu)-Ser(tBu)-Gly-Ala-Pro-Pro-Pro-Ser(tBu)-Rink amide MBHA resin.

**The specific coupling conditions are as follows:**

| **No.** | **Amino acid** | **Coupling conditions (DMF as the reaction solvent)** |
|---|---|---|
| 38 | Fmoc-Ser(tBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 39 | Fmoc-Pro-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 40 | Fmoc-Pro-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 41 | Fmoc-Pro-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 42 | Fmoc-Ala-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 43 | Fmoc-Gly-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 44 | Fmoc-Ser(tBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 45 | Fmoc-Ser(tBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 46 | Fmoc-Pro-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 47 | Fmoc-Gly-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 48 | Fmoc-Gly-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 49 | Fmoc-Ala-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 50 | Fmoc-Ile-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 51 | Fmoc-Leu-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 52 | Fmoc-Trp(Boc)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 53 | Fmoc-Gln(Trt)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 54 | Fmoc-Val-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 55 | Fmoc-Phe-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 56 | Fmoc-Ala-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 57 | Fmoc-Lys(Dde)-OH | Fmoc removal: 20% piperidine/DMF, 10 min × 2, washing 5 times with DMF; |
| | | Coupling: 2.0 eq. AA/2.4 eq. HOBt/2.4 eq. DIC/ 0.2 eq. DIPEA, 3 h, rt. |
| 58 | Fmoc-Gln(Trt)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1.5 h, rt. |
| 59 | Fmoc-Ala-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 60 | Fmoc-Ile-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 61 | Fmoc-Lys(Boc)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 62 | Fmoc-Asp(OtBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 63 | Fmoc-Leu-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 64 | Fmoc-Aib-OH | Fmoc removal: 20% piperidine/DMF, 10 min × 2, washing 5 times with DMF; |
| | | Coupling: 3.0 eq. AA/3.6 eq. HOBt/3.6 eq. DIC, 5 h, rt. |
| 65 | Fmoc-Ile -OH | Fmoc removal: 20% piperidine/DMF, 10 min × 2, washing 5 times with DMF; |
| | | Coupling: 3.0 eq. AA/3.6 eq. HOBt/3.6 eq. DIC, 8 h, rt. |
| 66 | Fmoc-Ser(tBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 67 | Fmoc-Tyr(tBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 68 | Fmoc-Asp(OtBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 69 | Fmoc-Ser(tBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 70 | Fmoc-Thr(tBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 71 | Fmoc-Phe-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 72 | Fmoc-Thr(tBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 73 | Fmoc-Gly-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 74 | Fmoc-Glu(OtBu)-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 75 | Fmoc-Aib-OH | Fmoc removal: 20% piperidine/DMF, 10 min × 2, washing 5 times with DMF; |
| | | Coupling: 3.0 eq. AA/3.6 eq. HOBt/3.6 eq. DIC, 8 h, rt. |
| 76 | Boc-Tyr(tBu) -OH | Fmoc removal: 20% piperidine/DMF, 10 min × 2, washing 5 times with DMF; |
| | | Coupling: 3.0 eq. AA/3.6 eq. HOBt/3.6 eq. DIC, 7 h, rt. |
| 77 | Fmoc-AEEA-OH | Dde removal: 2% hydrazine hydrate/DMF, 3 min × 2, washing 5 times with DMF; |
| | | Coupling: 3.0 eq. AA/3.6 eq. HOBt/3.6 eq. DIC, 1 h, rt. |
| 78 | Fmoc-AEEA-OH | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 79 | Fmoc-Glu-OtBu | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |
| 80 | 20-(tert-Butoxy)-20-oxoicosanoic acid | Fmoc removal: 20% piperidine/DMF, 10 min × 2, washing 5 times with DMF; |
| | | Coupling: 3.0 eq. AA/3.6 eq. HOBt/3.6 eq. DIC, 1 h, rt. |

Cleavage step: The cleavage reagent adopted in this technique was TFA/TIDPS/EDT/H₂O = 91/3/3/3, and the volume of the cleavage reagent was 8 times the weight of the resin. Cleavage was performed at room temperature for 2 h. The resin was removed by filtration. Methyl tert-butyl ether was added to the cleavage solution. The addition amount of the methyl tert-butyl ether was 8 times that of the cleavage solution (V/V). Precipitation and filtration were performed, and the filter cake was dried to finally obtain 29.0 g of a crude peptide product with a yield of 60.3%, a purity of 47.68%, and a content of 32.0%. (FIG. 3)

Through comparison between Example 1 and Comparative Example 1, it can be seen that the present disclosure adopts specially modified building block fragments to improve synthesis efficiency; the peptide resin synthesis is shortened from 43 steps to 37 steps, the yield is increased from 60% to 99.8%, and the purity is also nearly doubled. The present disclosure achieves a substantial increase in synthesis efficiency and purity only by adjusting the synthesis sequence and using the specially modified building blocks.

## Claims

1. A method for solid-phase synthesis of tirzepatide, comprising the following steps:
S1) employing an amide resin as the solid support, and adopting an Fmoc solid-phase synthesis strategy to sequentially couple Fmoc-protected amino acids or specially modified building blocks to afford a fully protected tirzepatide peptide resin; and
S2) subjecting the resulting fully protected tirzepatide peptide resin to cleavage, precipitation, filtration, and drying to afford tirzepatide;
wherein the sequential coupling of Fmoc-protected amino acids or specially modified building blocks comprises sequential coupling of Fmoc-Ser(tBu)-OH, Fmoc-Pro-OH, Fmoc-Pro-OH, Fmoc-Pro-OH, Fmoc-Ala-OH, Fmoc-Gly-OH, Fmoc-Ser(tBu)-OH, Fmoc-Ser(tBu)-OH, Fmoc-Pro-OH, Fmoc-Gly-OH, Fmoc-Gly-OH, Fmoc-Ala-OH, Fmoc-Ile-OH, Fmoc-Leu-OH, Fmoc-Trp(Boc)-OH, Fmoc-Gln(Trt)-OH, Fmoc-Val-OH, Fmoc-Phe-OH, Fmoc-Ala-OH, Fmoc-Lys(AEEA-AEEA-γ-Glu(α-OtBu)-eicosanedioic acid(mon-tBu))-OH, Fmoc-Gln(Trt)-OH, Fmoc-Ala-OH, Fmoc-Ile-OH, Fmoc-Lys(Boc)-OH, Fmoc-Asp(OtBu)-OH, Fmoc-Leu-OH, Fmoc-Ile-Aib-OH, Fmoc-Ser(tBu)-OH, Fmoc-Tyr(tBu)-OH, Fmoc-Asp(OtBu)-OH, Fmoc-Ser(tBu)-OH, Fmoc-Thr(tBu)-OH, Fmoc-Phe-OH, Fmoc-Thr(tBu)-OH, Fmoc-Gly-OH, Fmoc-Glu(OtBu)-OH, and Boc-Tyr-Aib-OH.

2. The method of Claim 1, wherein the Fmoc solid-phase synthesis strategy in step S1) comprises:
S11) removing an Fmoc protecting group; and
S12) coupling of Fmoc-protected amino acids or specially modified building blocks;
wherein in S11), 20% piperidine/DMF is employed as an Fmoc deprotection reagent; and said 20% piperidine/DMF is a mixed solution of piperidine and DMF at a volume ratio of 1:4;
the coupling the Fmoc-protected amino acids or specially modified building blocks in S12) is carried out in the presence of an amino acid or specially modified building block coupling agent.

3. The method of Claim 2, wherein deprotection in S11) comprises adding an Fmoc deprotection reagent and maintaining the reaction for 5-10 min, and washing with DMF, adding the Fmoc deprotection reagent again and maintaining the reaction for 5-10 min, and washing with DMF.

4. The method of Claim 2, wherein the coupling agent for the protected amino acids is a combination of DIC and A or a combination of DIPEA, A, and B, wherein A is selected from Oxyma, HOBt, or HOAt, and B is selected from the group consisting of PyBOP, PyAOP, HATU, HBTU, and TBTU; preferably, the coupling agent is a composition of DIC and HOBt.

5. The method of Claim 4, wherein upon coupling the protected amino acids, the coupling agent is used in a molar ratio of 1 to 1.5 equivalents relative to the protected amino acid.

6. The method of Claim 2, wherein the coupling agent for the specially modified building blocks selected from Fmoc-Lys(AEEA-AEEA-γ-Glu(α-OtBu)-eicosanedioic acid(mon-tBu))-OH, Fmoc-Ile-Aib-OH, and Boc-Tyr(tBu)-Aib-OH is a combination of DIC, HOBt and DIPEA.

7. The method of Claim 6, wherein upon coupling the specially modified building blocks, a molar ratio of the specially modified building block : DIC : HOBt : DIPEA is 1:(1-1.5):(1-1.5):(0.1-0.5),
preferably 1:1.2:1.2:0.1.

8. The method of Claim 1, wherein the amide resin in S1) is selected from the group consisting of Rink amide resin, Rink amide AM resin, Rink amide MBHA resin and Sieber resin;
preferably, the amide resin is Rink amide MBHA resin or Sieber resin;
preferably, the amide resin in S1) has a substitution level ranging fromsubstitution level 0.2 to 0.8 mmol/g.

9. The method of Claim 1, wherein the cleavage in S2) performed using a mixed solution of TFA, TIPS, EDT and H2O;
preferably, the volume ratio of TFA, TIPS, EDT and H2O is 91:3:3:3.

10. The method of Claim 1, wherein precipitation in S2) comprises adding an excess of methyl tert-butyl ether into the cleavage reaction mixture to effect precipitation;
preferably, the added methyl tert-butyl ether is 5-10 times the volume of the cleavage reaction mixture.
